# EUROPEAN PATENT SPECIFICATION

(11) **EP 0 678 331 B1**
(45) Date of publication and mention of the grant of the patent: **31.03.1999**
(21) Application number: 95104803.2
(22) Date of filing: 31.03.1995
(51) Int. Cl.: B01J 35/02, C07C 17/156, B01J 27/122

(54) **Catalyst in granular form for 1,2-dichloroethane synthesis and fixed-bed process for ethylene oxachlorination using said catalyst**
Katalysator in granulierter Form für die Synthese von 1,2-Dichloroethan und Verfahren zur Festbett Oxychlorierung von Ethylen unter Verwendung dieses Katalysators
Catalyseur sous forme granulaire pour la synthèse du 1,2-dichloroéthane et procédé d'oxychloration de l'éthylène en lit fixe utilisant ledit catalyseur

(30) Priority: 05.04.1994 IT MI940640
(43) Date of publication of application: 25.10.1995
(73) Proprietor: SÜD CHEMIE MT S.r.l., 20123 Milano (IT)
(72) Inventor: Viola, Augusto, I-28011 Armeno (NO) (IT); Cavalli, Luigi, I-28100 Novara (IT); Rossi, Michele, I-20068 Peschiera Borromeo (MI) (IT)
(74) Representative: Modiano, Guido, Dr.-Ing.

(56) References cited:
- EP-A- 0 375 202
- EP-A- 0 461 431
- EP-A- 0 464 633
- EP-A- 0 591 572
- DE-A- 3 113 179

## Description

The present invention relates to a supported catalyst in the form of cylindrical granules, for 1,2-dichloroethane synthesis by fixed-bed ethylene oxychlorination. More particularly, the present invention relates to a catalyst comprising CuCl₂ as its active compound, supported on an alumina carrier.

As known, the synthesis of 1,2-dichloroethane by oxidative chlorination of ethylene can be carried out in a fluidized-bed or fixed-bed reactor. In the first case, a more uniform temperature distribution is obtained inside the reactor (with the arising of localized overheating phenomena - - "hot spots"-- being prevented); unfortunately, at the expense of a certain difficulty of fluidization due to a tendency to sticking of the catalytic particles. In the second case, the reaction parameters control is simplier but owing to the reduced heat exchange coefficient between the catalyst granules, and between the latter and the reaction gas, hot spots may appear. The formation of such hot spots should be prevented for reasons of selectivity and useful catalyst life.

A first attempt to solve the problem of the heat exchange between the granules of catalyst for ethylene oxychlorination had made resort to ring-shaped granules or circular-cylindrical granules having a determined height-diameter ("aspect") ratio. Such a type of catalysts is disclosed, e.g., in EP-B-54674 and U.S. 4,753,914.

The problem of the heat exchange coefficient is not the only technical problem to be solved in the case of an efficient synthesis of 1,2-dichloethane in a fixed-bed reactor. In fact, a granular catalyst used in fixed-bed ethylene oxychlorination is also required to display the following characteristics:
-- low resistence to gas flow (low pressure drop with the height of catalytic bed being the same);
-- large actual specific surface area, i.e., high surface:volume ratio; and
-- good mechanical strength in order to prevent the catalytic particles from undergoing breakage with consequence bed packing.

The normally used catalysts in fixed-bed oxychlorination process (and having a spherical, solid-cylindrical, or ring shape, with different dimensions) do not solve the above said problems to a satisfactory extent. Furthermore, when such shapes known from the prior art are used, the diffusion of the reactant gases inside the interior of the catalyst granules and the back-diffusion of the reaction products from the interior of said granules result often to be very limited. This means that, inasmuch as in the heterogeneous system taken into consideration the oxychlorination reaction takes place more easily and selectively on the outer surface of catalyst granule, the oxychlorination catalysts having the shapes known from the prior art are not efficiently used. Therefore, in order to obtain the desired conversion rate, a large amount of catalyst must be used and therefore, in the case of tube-bundle fixed bed, tubes having suitable height must be used. With the known forms of oxychlorination catalysts, this causes a further increase in pressure drop, also because the empty spaces between the catalyst granules are limited.

Catalysts with different from traditional shapes are disclosed in U.S. patent No. 4,441,990; which relates to tubular extruded granules having an essentially triangular or quadrangular, multilobed cross section. With such catalyst, some advantages are achieved in terms of breakage strength and pressure drop, but the results are not really very much different from as obtainable with the traditional catalysts. Other non-traditional shapes of catalyst granules are disclosed in EP-A-464,633, with specific reference to the process of unsaturated esters production.

The purpose of the present invention is of supplying a novel oxychlorination catalyst which, besides considerably improving the obtainable results in terms of pressure drop, high surface area:volume ratio and high heat exchange coefficient as compared to the catalysts known from the prior art, makes it possible the activity and selectivity to dichloroethane of the reaction to be increased.

Such a purpose is achieved thanks to the cylindrical granule displaying at least three through-bores having axes substantially parallel to each other and to the axis of the granule, and substantially equidistant from each other.

The catalyst granules are preferably obtained by moulding alumina powder and subsequently impregnating the moulded bodies with aqueous solutions of CuCl₂ and KCl. Other alkali or alkaline-earth metal chlorides, e.g., MgCl₂, can be used in catalyst preparation.

Said through-bores preferably have a cross section of circular shape and, on the cross section of the particle, their axes define vertices of a substantially equilateral triangle, said vertices being oriented towards the points of contact of the cross section of the catalyst particle with the circumscribed circonference. According to the preferred embodiment of the invention, the granules display circular-cylindrical lobes equal to each other and coaxial with the through-bores.

Thanks to the above said characteristics, owing to the particular geometry of the granules, a high turbulence of the reaction gases on the same granules can be promoted under the same operating conditions as customarily adopted in the fixed-bed ethylene oxychlorination reactors. As they display a large free surface area in their cross section, said granules oppose a lower resistance to gas flow, with consequently lower pressure drops. Furthermore, their low equivalent diameter [wherein by the expression "equivalent diameter", the value calculated by: 6 · (volume/total area surface) is meant], results in a larger actual specific surface area, i.e., a high value of surface-area: volume ratio. This makes it possible a better contact of the reaction gases with the catalyst surface to be obtained, which favours the conversion of the reactants and limits the inner diffusion phenomena, with a consequent increase in oxychlorination reaction selectivity. In fact, with the catalyst according to the present invention, high yields of 1,2-dichloroethane are obtained by using a lower catalyst amount per unit volume, with respect to the catalysts having shapes known from the prior art.

According to a second embodiment of the present invention, the catalytic granule displays a cross-section substantially triangular shape with rounded vertices.

In both of the above embodiments, the ratio of the bore pitch (wherein, by "bore pitch", the distance between the respective axes is meant), to the diameter of the same bores, is preferably comprised within the range of from 1.15 to 1.5, and more preferably of from 1.3 to 1.4.

The ratio of the height of the particle to the bore pitch is preferably comprised within the range of from 1.5 to 2.5, more preferably of from 1.7 to 2.3.

According to the first embodiment, the ratio of the bending radius of each lobe to the bore pitch is preferably comprised within the range of from 0.6 to 0.9 and, more preferably, of from 0.7 to 0.8. The ratio of the bending radius of the lobes to the radius of the through-bores is preferably comprised within the range of from 1.3 to 2.7, more preferably of from 1.8 to 2.10. The ratio of the radius of the circumscribed circumference to the cross-section, to the bending radius of the circular lobes is preferably comprised within the range of from 1.6 to 2, more preferably of from 1.7 to 1.85. The ratio of the surface area to the volume of each granule in the multilobed modification results to be preferably higher than 2.0, more preferably higher than 2.2.

According to the second embodiment of the present invention, the ratio of the bending radius of each rounded vertex to the bore pitch is preferably comprised within the range of from 0.6 to 0.9, and, more preferably, of from 0.7 to 0.8. The ratio of the radius of the circumscribed circumference to the cross section, to the bending radius of each rounded vertex is preferably comprised within the range of from 1.6 to 2, and, more preferably, of from 1.7 to 1.85. The ratio of the surface area to the volume of each granule in the multilobed modification results to be preferably higher than 2.0 and, still more preferably, higher than 2.2.

A further object of the present invention is a process for fixed-bed oxidative chlorination of ethylene into 1,2-dichloroethane which uses a catalyst having the novel shape as defined above. The fixed-bed modification of ethylene oxychlorination process normally implies using a plurality of reactors in cascade consisting of suitably thermostated bundles of catalyst containing tubes. The reactor feed is generally constituted by a gas mixture of ethylene, hydrogen chloride and air. According to a modification of the fixed-bed oxychlorination process, air is replaced by oxygen.

In the fixed-bed oxychlorination process, the shape and size of catalyst granule result to be of basic importance. By means of a granule having the geometry according to the present invention, surprising advantages are attained in terms of activity, selectivity, heat exchange and pressure drop through the catalytic bed.

The particular geometry of the oxychlorination catalyst granule according to the present invention is disclosed now in detail, for merely exemplifying, non-limitative purposes, by referring to the accompaning drawings, in which:
-- Figure 1 is a plan view of a first embodiment of a catalytic granule for ethylene oxychlorination according to the present invention, and
-- Figure 2 is a plan view of a second embodiment of the catalytic granule according to the present invention.

Referring to the drawings, with 10 a cylindrical granule (pellet) of catalyst for oxychlorination is displayed, which is provided with three circular through-bores 12 arranged with their respective centres at the vertices of an equilateral triangle.

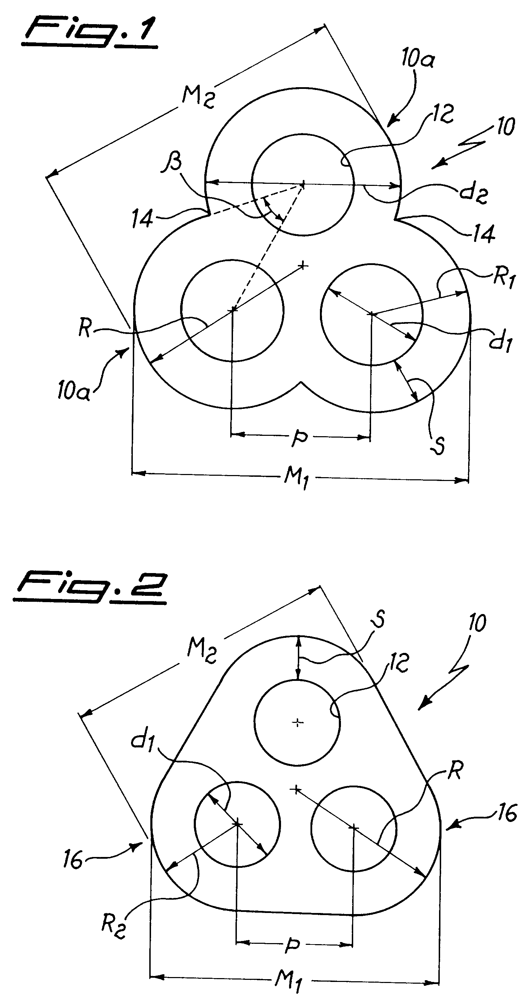

According to the embodiment illustrated in Figure 1, the pellet displays a trilobed cross-section, with circular lobes 10a joining each other at longitudinal grooves 14 arranged along the side surface of the pellet. The bores 12, the diameter of which is indicated in the drawings with the reference character d₁, are coaxial with the circular lobes 10a and together with them define walls of thickness "s". With "p", the pitch between the bores 12 (i.e., the distance between their centres) is indicated, and with d₂, the diameter of lobes 10a is indicated (the radius of said lobes is indicated with R₁). The radius of circumscribed circumference to the cross section of the cylindrical pellet is indicated with the reference R. With M₁ and M₂, the maximal and minimal dimensions of the cross section of the pellet are indicated.

Referring to the embodiment illustrated in Figure 2, and in which, for analogous parameters, the same reference numerals and characters are used as in Figure 1, the catalyst pellet displays a triangular cross section with rounded vertices 16. The latter have a bending radius as indicated with R₂.

In Tables 1, 2 and 3 enclosed with the instant disclosure, the size parameters are respectively reported of oxychlorination catalyst pellets according to Figures 1 and 2, and of a type of traditional catalyst pellet of ring-like shape ("A" pellets), manufactured by using the fabrication technology as disclosed in the following examples. The physical-chemical characterizations of the catalysts are reported in Table 4.

From the data relevant to the dimensions and shape of the catalytic pellets, the volume of the solid body corresponding to the shape of one single pellet ("volume of corresponding solid body") of each pellet; and, from it, by measuring the bulk density of the catalyst (which depends on the fabrication pressure, on the characteristics of the alumina used as the starting material and on the firing modalities), the expected weight for each pellet can be calculated. The expected weight so calculated is in accordance with the experimentally found weight throughout the tested range of equivalent diameter values (3.50 - 2.20 mm).

The activity, selectivity rate and pressure drop values through the catalytic bed are reported in Table 5.

### EXAMPLES 1-4

A bohemite alumina powder having a surface area of 270 m²/g and a pore volume of 0.5 cm³/g was pelletized in order to yield shaped bodies as indicated in Table 1, and precisely cylindrical bodies of conventional type of 5x5x2 mm (Example A) and having the novel geometries according to the present invention (Examples B, C and D), by using suitable forming moulds.

After firing at 550°C for 3 hours, the pellets were impregnated with a solution containing copper dichloride and potassium chloride in such amounts as to yield the following composition, by weight: CuCl₂ = 15%; KCl = 5%; Al₂O₃ = 80%. After impregnation, the pellets were submitted to drying at 150°C for 3 hours.

In order to determine the activity, the yield and the pressure drop of the catalysts, a tubular reactor of nickel was used which had an internal diameter of 26.6 mm and a height of 1300 mm, vertically installed inside a silicone oil based thermostating bath. The catalyst was charged to the fixed-bed tubular reactor by using the following charging profile, from up downwards:
-- a 1st layer of 400 mm of thickness, constituted by catalyst mixed with graphite, having the form of extruded cylindrical bodies of 5x5 mm, in the ratio of catalyst:diluent = 1:1 by volume;
-- a 2nd layer of 400 mm of thickness, constituted by neat catalyst.

Through the reactor a gas stream was fed from up downwards, with the following volume rate:
* ethylene = 21.6 Nl/h;
* HCl = 40 Nl/h;
* air = 57 Nl/h.

The external thermostating bath was kept at a temperature which secured an HCl conversion of 99%. The pressure downstream from the reactor was of 1 atm, and the reactor-head pressure compensated for the pressure drop through the reactor (ΔP).

The reactor exiting reaction products were quenched. The liquid fraction was analyzed by gaschromatography by using a Hewlett-Packard gaschromatograph with capillary column suitable for separating 1,2-dichloroethane, chloral, ethyl chloride and other chlorinated byproducts, and the gas fraction was analyzed by means of a Carlo Erba model Fractovap gas chromatograph equipped with suitable columns for separating ethylene, CO, CO₂, O₂ and N₂.

By comparing the results, it can be clearly inferred that lower pressure drops are obtained with the catalyst shape according to the present invention, by using the same catalyst volume. If one takes into consideration that this novel catalyst displays a lower bulk density (expressed as g/cm³), the advantage results to be still greater. In particular, the catalysts display a high HCl conversion at same, or lower, temperatures, as well as a higher selectivity (Examples B, C and D).

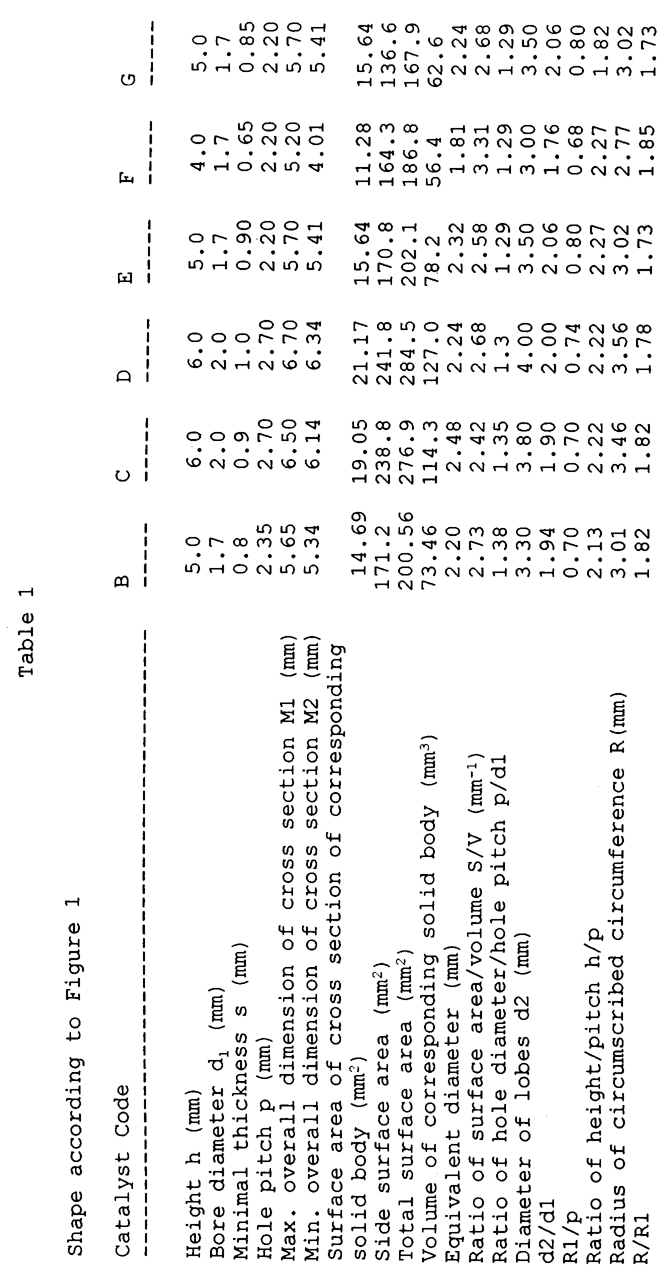

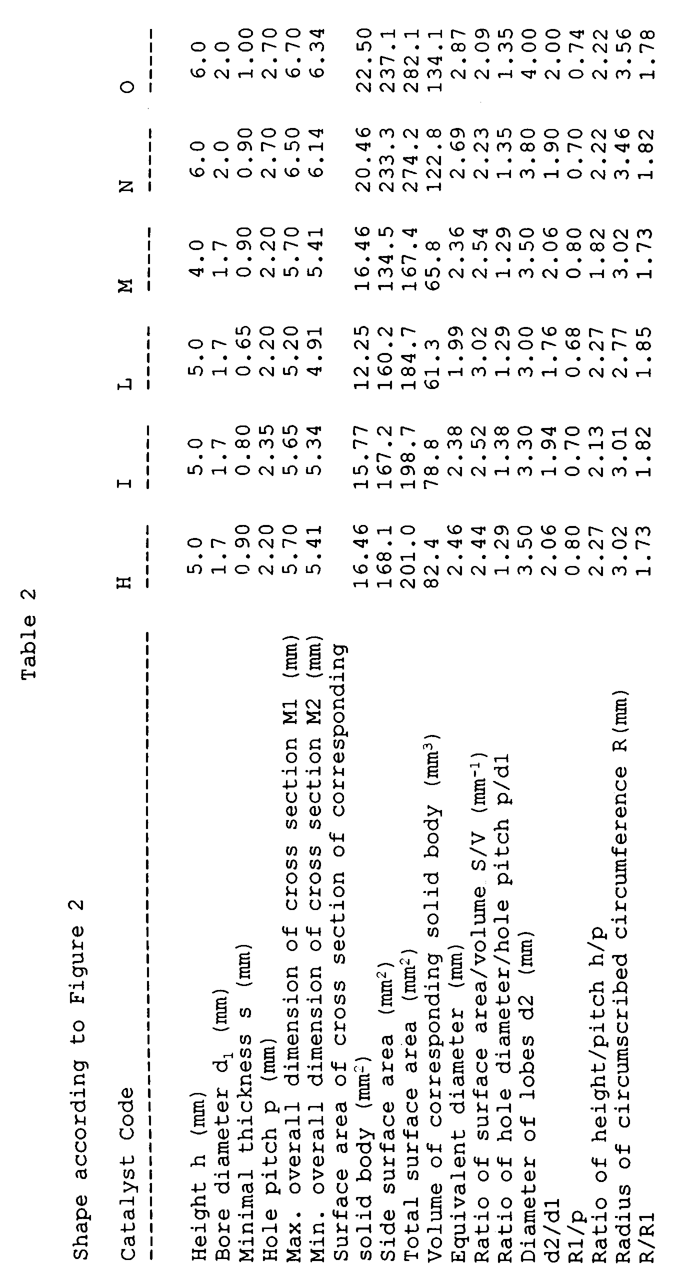

**TABLE 3**

| Cylindrical shape | |
|---|---|
| Catalyst code | A |
| Height (mm) | 5.0 |
| Outer diameter (mm) | 5.0 |
| Inner diameter (mm) | 2.0 |
| Thickness (mm) | 1.5 |
| Surface area of cross section of corresponding solid body (mm²) | 16.5 |
| Side surface area (mm²) | 110.0 |
| Total surface area (mm²) | 143 |
| Volume of corresponding solid body (mm³) | 82.5 |
| Equivalent diameter (mm) | 3.5 |
| Free surface area of cross section (mm²) | 3.1 |

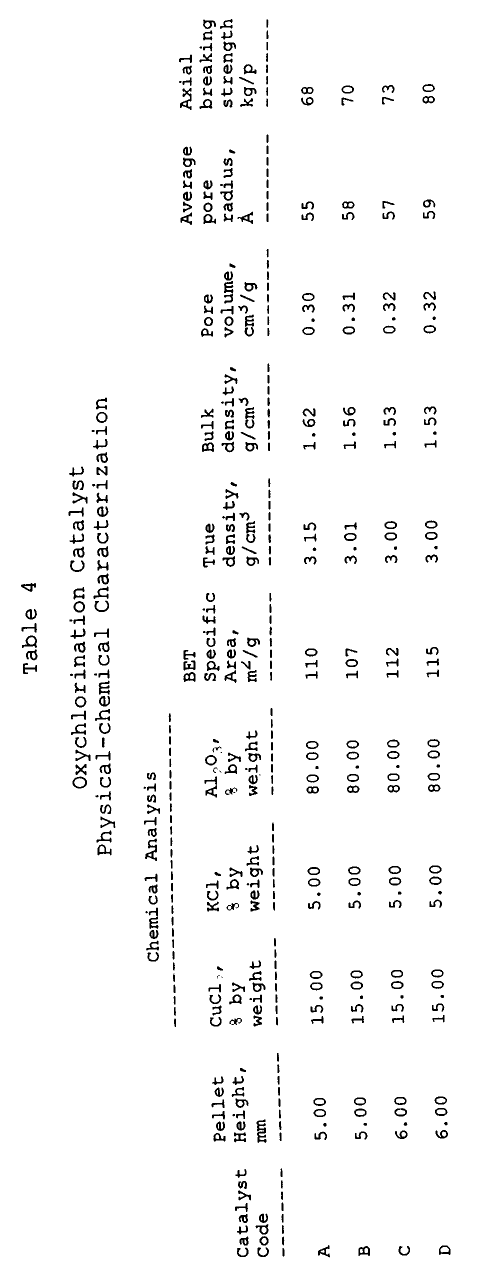

**TABLE 5**

| |
|---|
| Inner diameter of the reactor = 26.6 mm |
| Height of the catalytic bed = 800 mm |

| Flow rates: |
|---|
| Ethylene = 21.6 Nl/h |
| HCl = 40 Nl/h |
| Air = 57 Nl/h |

| Catalyst composition: |
|---|
| CuCl₂ = 15% by weight |
| KCl = 5% by weight |
| Al₂O₃ = 80% by weight |

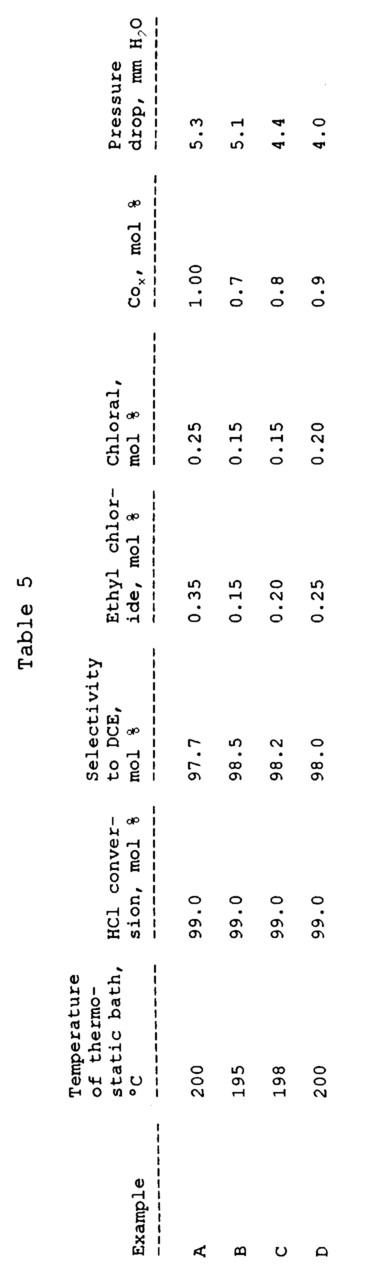

## Claims

1. Supported catalyst having the form of cylindrical granules for 1,2-dichloroethane synthesis by fixed-bed oxychlorination of ethylene, comprising copper chloride as its active component, characterized in that the catalyst granules display at least three through-bores having axes which are substantially parallel to each other and to the axes of the granule, and substantially equidistant from each other.

2. Catalyst according to claim 1, characterized in that said granules are obtained by moulding alumina powder and subsequently impregnating the resulting moulded bodies with copper chloride and potassium chloride.

3. Catalyst according to claim 1 or 2, characterized in that said through-bores have a circular cross section and have axes which, on the cross section of the particle, define vertices of a substantially equilateral triangle, with said vertices being orientated towards the point of contact of the cross section with the circumscribed circumference.

4. Catalyst according to claim 3, characterized in that said granules display circular-cylindrical lobes, equal to each other and coaxial with said through-bores.

5. Catalyst according to claim 3, characterized in that the ratio of the bore pitch (p) to the diameter of the bores (d₁) is comprised within the range of from 1.15 to 1.5.

6. Catalyst according to claim 3, characterized in that the ratio of the height of the particle to the pitch of the bores is comprised within the range of from 1.5 to 2.5.

7. Catalyst according to claim 3, characterized in that the ratio of the bending radius of each lobe to the pitch of the bores is comprised within the range of from 0.6 to 0.9.

8. Catalyst according to claim 7, characterized in that the ratio of the bending radius of the lobes to the radius of the through-bores is comprised within the range of from 1.3 to 2.7.

9. Catalyst according to claim 3, characterized in that the ratio of the radius of the circumscribed circumference to the cross section, to the bending radius of each rounded vertex is preferably comprised within the range of from 1.6 to 2.

10. Catalyst according to claim 3, characterized in that the ratio of the surface area to the volume of each granule is higher than 2.

11. Process for oxidative chlorination of ethylene into 1,2-dichloroethane, in which a stationary bed of cylindrical granules of supported, copper chloride-based catalyst is used, characterized in that each granule displays at least three through-bores having substantially parallel axes to each other and to the axes of the granule, and substantially equidistant from each other.

12. Process according to claim 11, characterized in that said catalyst granules display lobes of circular-cylindrical shape equal to each other and coaxial with said through-bores.

## Patentansprüche

1. Trägergestützter Katalysator mit der Form von zylindrischen Körnchen für die 1,2-Dichlorethansynthese durch Festbett-Oxychlorierung von Ethylen, umfassend Kupferchlorid als seine aktive Komponente, dadurch gekennzeichnet, daß die Katalysatorkörnchen mindestens drei durchgehende Bohrungen aufweisen, die im wesentlichen parallele Achsen zueinander und zu der Achse des Körnchens aufweisen und zueinander im wesentlichen gleich beabstandet sind.

2. Katalysator nach Anspruch 1, dadurch gekennzeichnet, daß die Körnchen durch Formen von Aluminiumoxidpulver und anschließendes Imprägnieren der erhaltenen Formkörper mit Kupferchlorid und Kaliumchlorid erhalten werden.

3. Katalysator nach Anspruch 1 oder 2, dadurch gekennzeichnet, daß die durchgehenden Bohrungen einen kreisförmigen Querschnitt aufweisen und Achsen besitzen, die auf dem Querschnitt der Teilchen Scheitelpunkte eines im wesentlichen gleichseitigen Dreiecks definieren, wobei die Scheitelpunkte zu dem Kontaktpunkt des Querschnitts mit dem umzeichneten Umfang ausgerichtet sind.

4. Katalysator nach Anspruch 3, dadurch gekennzeichnet, daß die Körnchen kreisförmig-zylindrische Ausbuchtungen aufweisen, die zueinander gleich und koaxial mit den durchgehenden Bohrungen sind.

5. Katalysator nach Anspruch 3, dadurch gekennzeichnet, daß das Verhältnis des Zwischenraumabstandes (p) der Bohrung zu dem Durchmesser (d₁) der Bohrungen im Bereich 1,15 bis 1,5 liegt.

6. Katalysator nach Anspruch 3, dadurch gekennzeichnet, daß das Verhältnis der Höhe der Teilchen zu dem Zwischenraumabstand der Bohrungen im Bereich 1,5 bis 2,5 liegt.

7. Katalysator nach Anspruch 3, dadurch gekennzeichnet, daß das Verhältnis des Krümmungsradius einer jeden Ausbuchtung zu dem Zwischenraumabstand der Bohrungen im Bereich 0,6 bis 0,9 liegt.

8. Katalysator nach Anspruch 7, dadurch gekennzeichnet, daß das Verhältnis des Krümmungsradius der Ausbuchtungen zu dem Radius der durchgehenden Bohrungen im Bereich 1,3 bis 2,7 liegt.

9. Katalysator nach Anspruch 3, dadurch gekennzeichnet, daß das Verhältnis des Radius des umzeichneten Umfangs für den Querschnitt zu dem Krümmungsradius jedes abgerundeten Scheitels vorzugsweise im Bereich 1,6 bis 2 liegt.

10. Katalysator nach Anspruch 3, dadurch gekennzeichnet, daß das Verhältnis der Oberfläche zu dem Volumen von jedem Körnchen größer als 2 ist.

11. Verfahren zur oxidativen Chlorierung von Ethylen zu 1,2-Dichlorethan, wobei ein Festbett von zylindrischen Körnchen eines trägergestützten Katalysators auf Kupferchloridbasis verwendet wird, dadurch gekennzeichnet, daß jedes Körnchen mindestens drei durchgehende Bohrungen aufweist, die im wesentlichen parallele Achsen zueinander und zu der Achse des Körnchens aufweisen und zueinander im wesentlichen gleich beabstandet sind.

12. Verfahren nach Anspruch 11, dadurch gekennzeichnet, daß die Katalysatorkörnchen Ausbuchtungen von kreisförmig-zylindrischer Form aufweisen, die gleich zueinander und koaxial mit den durchgehenden Bohrungen sind.

## Revendications

1. Catalyseur supporté présentant la forme de granulés cylindriques pour la synthèse du 1,2-dichloroéthane par oxychloration de l'éthylène en lit fixe, comprenant du chlorure de cuivre en tant que composant actif, caractérisé en ce que chaque granulé présente au moins trois trous traversants dont les axes sont sensiblement parallèles les uns aux autres et aux axes du granulé, et sensiblement équidistants les uns par rapport aux autres.

2. Catalyseur selon la revendication 1, caractérisé en ce que lesdits granulés sont obtenus par moulage de poudre d'alumine et par imprégnation subséquente des corps moulés obtenus à l'aide de chlorure de cuivre et de chlorure de potassium.

3. Catalyseur selon les revendications 1 ou 2, caractérisé en ce que lesdits trous traversants présentent une section transversale circulaire et présentent des axes qui sur la section transversale de la particule, définissent des sommets d'un triangle sensiblement équilatéral, lesdits sommets étant orientés vers le point de contact de la section transversale avec le cercle circonscrit.

4. Catalyseur selon la revendication 3, caractérisé en ce que lesdits granulés présentent des lobes cylindraux circulaires, égaux les uns aux autres et coaxiaux avec lesdits trous traversants.

5. Catalyseur selon la revendication 3, caractérisé en ce que le rapport entre l'écartement des trous (p) et le diamètre des trous (d₁) est compris dans la fourchette de 1,15 à 1,5.

6. Catalyseur selon la revendication 3, caractérisé en ce que le rapport entre la hauteur des particules et l'écartement des trous est compris dans la fourchette de 1,5 à 2,5.

7. Catalyseur selon la revendication 3, caractérisé en ce que le rapport entre le rayon de courbure de chaque lobe et l'écartement des trous est compris dans la fourchette de 0,6 à 0,9.

8. Catalyseur selon la revendication 7, caractérisé en ce que le rapport entre le rayon de courbure des lobes et le rayon des trous traversants est compris dans la fourchette de 1,3 à 2,7.

9. Catalyseur selon la revendication 3, caractérisé en ce que le rapport entre le rayon de la circonférence circonscrit à la section transversale, et le rayon de courbure de chaque sommet arrondi est de préférence compris dans la fourchette de 1,6 à 2.

10. Catalyseur selon la revendication 3, caractérisé en ce que le rapport entre l'aire de surface et le volume de chaque granulé est supérieur à 2.

11. Procédé de chloration oxydante de l'éthylène en 1,2-dichloroéthane dans lequel on utilise un lit stationnaire de granulés cylindriques de catalyseur supporté à base de chlorure de cuivre, caractérisé en ce que chaque granulé présente au moins trois trous traversants ayant des axes sensiblement parallèles les uns aux autres et aux axes du granulé et sensiblement équidistants les uns des autres.

12. Procédé selon la revendication 11, caractérisé en ce que lesdits granulés de catalyseur présentent des lobes de forme circulaire cylindrique égaux les uns aux autres et coaxiaux avec lesdits trous traversants.
